# EUROPÄISCHE PATENTSCHRIFT

(11) **EP 0 975 761 B1**
(45) Veröffentlichungstag und Bekanntmachung des Hinweises auf die Patenterteilung: **14.01.2004**
(21) Anmeldenummer: 98920533.1
(22) Anmeldetag: 16.04.1998
(51) Int. Cl.: C12N 15/22, C07K 14/565, A61K 38/21

(54) **HUMANES, REKOMBINANTES INTERFERON-BETA MIT VERBESSERTER LÖSLICHKEIT**
RECOMBINANT HUMAN BETA INTERFERON WITH ENHANCED SOLUBILITY
INTERFERON-BETA HUMAIN RECOMBINE A SOLUBILITE AMELIOREE

(30) Priorität: 23.04.1997 DE 19717864
(43) Veröffentlichungstag der Anmeldung: 02.02.2000
(73) Patentinhaber: Fraunhofer-Gesellschaft zur Förderung der angewandten Forschung e.V., 80686 München (DE)
(72) Erfinder: SCHNEIDER-FRESENIUS, Christian, D-30177 Hannover (DE); OTTO, Bernd, D-30659 Hannover (DE); WASCHÜTZA, Gero, D-38536 Meinersen (DE)
(86) Internationale Anmeldenummer: PCT/EP1998/002238
(87) Internationale Veröffentlichungsnummer: WO 1998/048018

(56) Entgegenhaltungen:
- EP-A- 0 163 993

## Beschreibung

Die Erfindung betrifft Varianten des humanen, rekombinanten Interferon-β mit verbesserter Löslichkeit.

Interferon-β ist ein regulatorisches Protein, das durch Rezeptorbindung zur Aktivierung von Genen führt. Dadurch werden in der Zelle antivirale, antiproliferative sowie weitere biologische Aktivitäten vermittelt.

Interferone gehören, wie die Interleukine, zur Klasse der Cytokine und gliedern sich in unterschiedliche Klassen:
Interferon Typ I (-α,-β,-ω,-τ) und Typ II (-γ)

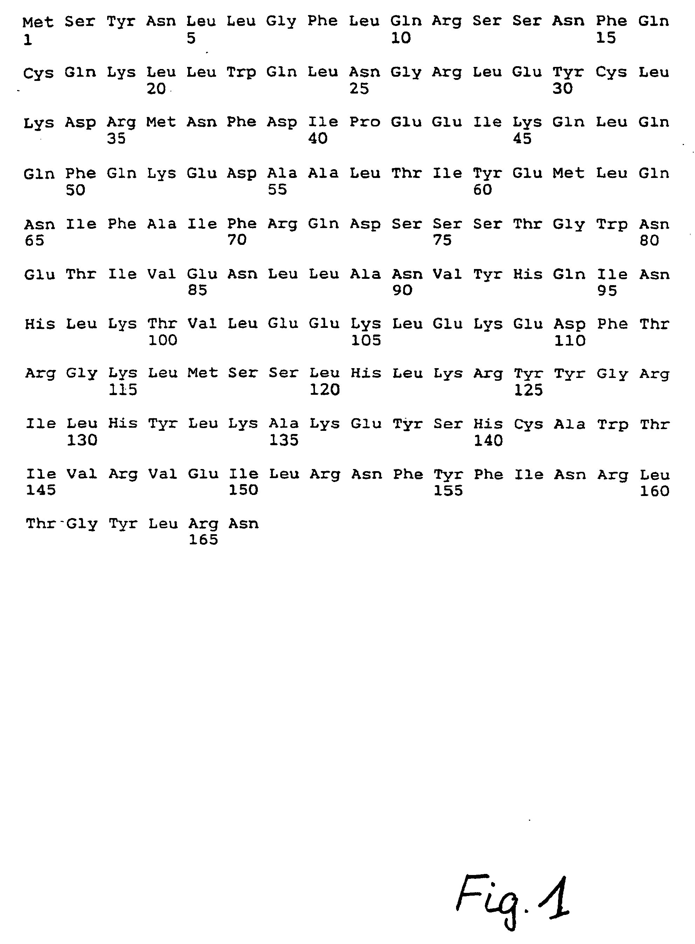

Humanes Interferon-β ist ein Protein mit einem Molekulargewicht von 22 kDa und 166 Aminosäure-Resten. Es wird hauptsächlich in Fibroblasten bei Virusbefall gebildet und besitzt antivirale, antiproliferative sowie weitere biologische Aktivitäten. Die Aminosäuresequenz des humanen Interfernon-β wurde erstmals von Taniguchi et al. (1980), Gene Bd. 10, Seiten 11 bis 15, veröffentlicht und ist in Fig. 1 dargestellt.

Gentechnologisch erzeugtes Interferon-β aus Bakterienoder Säugerzellen wird erfolgreich bei der Behandlung der Multiplen Sklerose eingesetzt, einer bislang unheilbaren Krankheit mit großem Patientenkollektiv. Als problematisch für die Produktion und Aufarbeitung des rekombinanten humanen Interferon-β erweist sich jedoch die sehr hohe Hydrophobizität des Proteins, die eine sehr schlechte Löslichkeit des rekombinanten humanen Interferon-β verursacht.

Aufgabe der vorliegenden Erfindung ist es, Varianten des rekombinanten humanen Interferon-β zur Verfügung zu stellen, deren Löslichkeit in polaren Medien wie beispielsweise wässrigen Flüssigkeiten verbessert ist. weiterhin ist es Ziel dieser Erfindung, Herstellungsverfahren für und Möglichkeiten der Verwendung von Varianten des rekombinanten humanen Interferon-β mit erhöhter Löslichkeit in polaren Medien wie wässrigen Flüssigkeiten anzugeben.

Diese Aufgabe wird durch das rekombinante humane Interferon-β gemäß Anspruch 1, seine Verwendung gemäß Anspruch 5 sowie seine Herstellung gemäß Anspruch 6 oder 7 gelöst.

Gemäß Anspruch 1 wird in dem bekannten humanen Interferon-β mindestens eine der folgenden zehn hydrophoben Aminosäuren gegen eine hydrophile Aminosäure ausgetauscht: Leu 5, Phe 8, Phe 15, Leu 47, Phe 50, Leu 106, Phe 111, Leu 116, Leu 120 oder Phe 156. Die Erfindung bezieht sich dabei auf die Einzelmutationen sowie alle möglichen Kombinationen dieser einzelnen Aminosäuren-Austausche.

Die genannten Aminosäuren liegen im wesentlichen an der Oberfläche des humanen Interferon-β und nehmen dort einen relativ großen Anteil der Oberfläche ein. Der Austausch dieser Aminosäuren führt daher zu einer überproportional großen Verbesserung des hydrophilen Charakters der Oberfläche des rekombinanten humanen Interferon-β und erhöht daher die Löslichkeit dieses Proteins in polaren Medien wie zum Beispiel wässrigen Flüssigkeiten. Das erfindungsgemäße rekombinante humane Interferon-β ist aufgrund seiner erhöhten Hydrophilität in der Produktion sowie in der Aufarbeitung zu einem Wirkstoff erheblich einfacher handzuhaben.

Die Produktion der erfindungsgemäßen Varianten des rekombinanten humanen Interferon-β erfolgt in allgemein bekannter, herkömmlicher Weise mit Hilfe von Mikroorganismen beispielsweise in einer mit dem Gen für eines der erfindungsgemäßen Proteine versehenen Escherichia coli - Kultur. Die Herstellung dieser gentechnisch veränderten Mikroorganismen erfolgt ebenfalls in allgemein bekannter Weise mit Hilfe klassischer gentechnischer Mutageneseverfahren zum Austausch der entsprechenden Aminosäuren gegen hydrophile Aminosäuren, auf deren Darstellung an dieser Stelle daher verzichtet wird.

Die erfindungsgemäßen Proteine finden Anwendung zur Herstellung von Arzneimitteln, beispielsweise gegen Multiple Sklerose, sowie als Feinchemikalie für In vitro-Versuche oder für Interferonspiegelmessungen. Die verbesserte Hydrophilität dieser Proteine vereinfacht dabei sowohl die Herstellung, den Transport, die Aufbewahrung als auch die Applikation als Arzneimittel oder Feinchemikalie.

Vorteilhafte Weiterbildungen der erfindungsgemäßen Proteine werden in den abhängigen Ansprüchen gegeben.

Besonders vorteilhaft ist der Austausch gegen die Aminosäuren Serin, Tyrosin und Threonin, die mit je einer Hydroxygruppe besonders hydrophil sind.

Serin eignet sich aufgrund seiner geringen Größe besonders für einen Austausch, da mit ihm besonders geringe sterische Änderungen des Proteins verbunden sind.

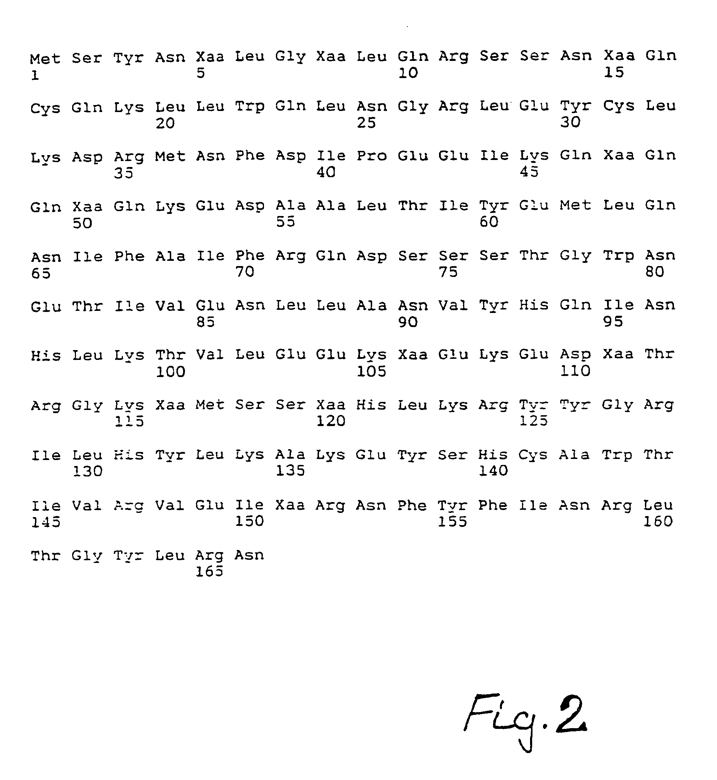

In Fig. 2 ist die Aminosäuresequenz des nativen rekombinanten humanen Interferon-ß dargestellt, bei dem die obengenannten zehn Aminosäuren wahlweise gegen Serin ausgetauscht werden. Diese austauschbaren Aminosäuren sind als Xaa dargestellt. Werden diese Aminosäuren ausgetauscht, so wird die Hydrophilität der Oberfläche des rekombinanten humanen Interferon-β sehr stark verbessert, während lediglich geringe Beeinträchtigungen der Funktionalität und Wirksamkeit des humanen rekombinanten Interferon-β auftreten.

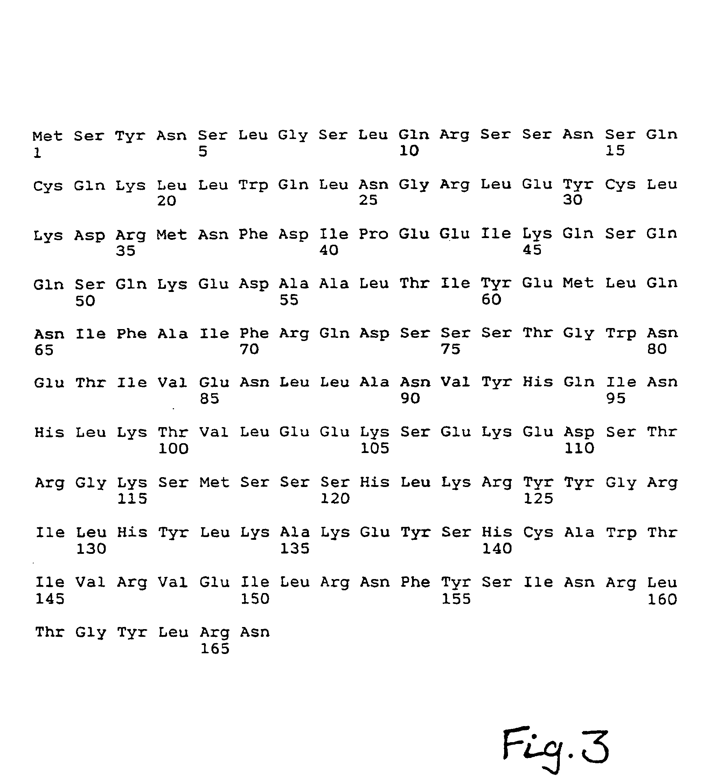

Eine besonders vorteilhafte Weiterbildung ist in Fig. 3 dargestellt, wobei hier sämtliche der obengenannten zehn Aminosäuren gegen Serin ausgetauscht wurden, so daß sich eine besonders starke Erhöhung der Hydrophilität der Oberfläche des rekombinanten humanen Interferon-β ergibt.

Im folgenden wird ein Beispiel für eine erfindungsgemäße Variante des humanen rekombinanten Interferon-β gegeben:
- Fig. 1: zeigt das native humane rekombinante Interferon-β,
- Fig. 2: zeigt die erfindungsgemäßen Variantendes rekombinanten humanen Interferon-β,
- Fig. 3: zeigt ein erfindungsgemäßes rekombinantes humanes Interferon-β, und
- Fig. 4: zeigt Primer für die Mutagenese für die Herstellung erfindungsgemäßen Interferon-β.

Wie bereits oben beschrieben, ist in Fig. 1 das native humane rekombinante Interferon-β beschrieben, wie es bereits jetzt mit Hilfe der bekannten molekularbiologischen und biotechnischen Möglichkeiten hergestellt werden kann.

In Fig. 2 ist die Sequenz des nativen humanen rekombinanten Interferon-β dargestellt, wobei mit Xaa diejenigen Aminosäuren bezeichnet wurden, die gegen eine Aminosäure mit mindestens einer Hydroxygruppe, vorteilhafterweise gegen Serin, Tyrosin und/oder Threonin ausgetauscht werden können und dabei ein erfindungsgemäßes rekombinantes humanes Interferon-β ergeben, das eine erhöhte Oberflächenhydrophilität besitzt.

Beispielsweise wurden sämtliche zehn Einzelvarianten des Interferon-β, bei denen Leu (5), Phe (8), Phe (15), Leu (47), Phe (50), Leu (106), Phe (111), Leu (116), Leu (120) oder Phe (156) gegen Serin ausgetauscht wurde, hergestellt, wobei sich insbesondere die Varianten mit dem Austausch der Aminosäuren Leu (5), Phe (8), Leu (47), Phe (50), Leu (106), Phe (111), Leu (116), Leu (120) gegenüber dem nativen Interferon-β, sowie auch diese Varianten der Cys-17-Ser-Variante des humanen Interferon-β durch eine vergleichbare biologische Aktivität auszeichnen.

Fig. 3 zeigt ein Beispiel für ein humanes rekombinantes Interferon-β, bei dem die folgenden Aminosäuren gegen Serin ausgetauscht wurden:

Leu(5), Phe(8), Phe(15), Leu(47), Phe(50), Leu(106), Phe(111), Leu(116), Leu(120) sowie Phe(156). Bei dieser Variante des rekombinanten humanen Interferon-β ergibt sich eine besonders hohe Hydrophilität der Oberfläche des Proteins und damit eine besonders gute Löslichkeit in wässrigen Lösungen.

Beispielsweise zeigt auch die Mehrfachvariante mit Aminosäureaustauschen an den Positionen 47, 50, 106, 111, 116 und 120 eine dem in diesem Beispiel verwendeten Ausgangsprotein Interferon-β mit einem Cys17Ser-Austausch entsprechende Aktivität.

Die Herstellung von Organismen mit einem erfindungsgemäß veränderten Gen für humanes rekombinantes Interferon-β erfolgt mittels klassischer Mutagenese-Verfahren.

Die Mutagenese erfolgt mittels PCR (Polymerasekettenreaktion). Die Mutationen werden durch synthetische Oligonukleotide eingeführt. Als Templat dient Plasmid-DNA mit dem IFN-β-Gen. Bei der hier angewandten PCR-Methode wird das gesamte Plasmid vervielfältigt. Die Selektion der PCR-Fragmente erfolgt durch einen Restriktionsverdau des gesamten PCR-Ansatzes mit dem Enzym DpnI. Dieses Enzym erkennt nur methylierte Schnittstellen. Da in-vitro durch PCR erzeugte Fragmente unmethyliert sind, wird durch DpnI nur die Templat-DNA abgebaut. Im Erfolgsfall bleibt nach dem DpnI-Verdau ein Fragment von der Länge des linearisierten Templates übrig, das die Mutationen trägt. Die erzeugten PCR-Fragmente werden kloniert und sequenziert.

| | | |
|---|---|---|
| PCR-Ansatz (100 µl) | Templat-DNA | 10 µg |
| | Primer | je 100 pmol |
| | Nukleotidmix | 200 µM je dNTP |
| | MgSO₄ | 2-6 mM |
| | DNA-Polymerase | 2,5 Units |

| PCR-Protokoll | Dauer | Temperatur |
|---|---|---|
| Schritt 1. | 4 min | 95°C |
| Schritt 2. | +Enzym | |
| Schritt 3. | 45 sec | 95°C |
| Schritt 4. | 1 min | 55°C |
| Schritt 5. | 10 min | 68°C . goto 3. (11x) |
| Schritt 6. | 10 min | 68°C |
| Schritt 7. | | 4°C |

Die PCR wurde auf einem Thermocycler PTC-200 (Fa MJ Research) durchgeführt.

Für jede PCR sind zwei Primer notwendig, ein "forward"und ein "reverse"-Primer. Beide Primer schließen sich direkt aneinander an, binden aber an jeweils unterschiedlichen Strängen und mit entgegengesetzter Orientierung.

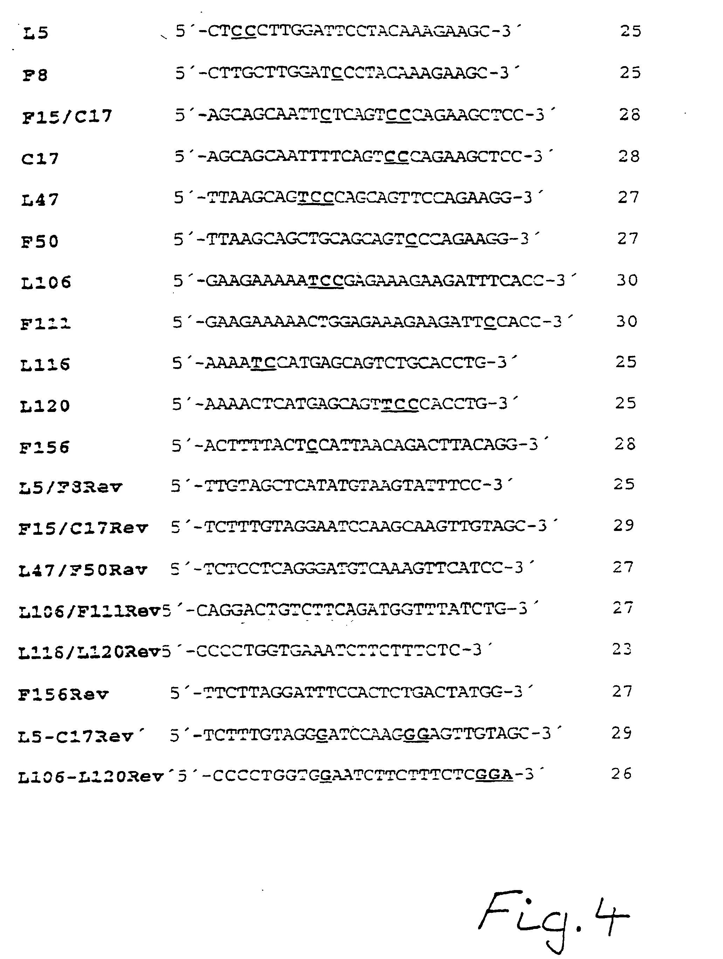

In Fig. 4 sind Primer dargestellt, die für die Mutagenese verwendet wurden, damit im Genprodukt, dem erfindungsgemäßen Interferon-β, die linksseitig benannte Aminosäure(n) gegen Serin ausgetauscht sind.

Die letzten beiden Primer enthalten als einzige "reverse"-Primer Mutationen. Eine PCR mit diesen Primern führt zusätzlich Mutationen ein, ohne die zuvor eingefügten Mutationen 5/8 bzw. 106/111 wieder aufzuheben. Wird das Wildtypgen als Templat genommen, lassen sich mit den "reverse"-Primern vier Mutationen auf einmal einführen (5-17 bzw. 106-120).

## Patentansprüche

1. Humanes, rekombinantes Interferon-β,
**dadurch gekennzeichnet,**
**daß** mindestens eine der Aminosäuren Leu(5), Phe(8), Phe(15), Leu(47), Phe(50), Leu(106), Phe(111), Leu(116), Leu(120), Phe(156) gegen Aminosäuren mit mindestens einer Hydroxy-Gruppe ausgetauscht ist.

2. Interferon-β nach Anspruch 1, **dadurch gekennzeichnet, daß** die Aminosäuren mit Hydroxygruppen Serin, Tyrosin und/oder Threonin' sind.

3. Interferon-β nach mindestens einem der vorhergehenden Ansprüche, **dadurch gekennzeichnet, daß** es folgende Aminosäuresequenz enthält: wobei eine oder mehrere der Aminosäuren Xaa(5), Xaa(8), Xaa(15), Xaa(47), Xaa(50), Xaa(106), Xaa(111), Xaa(116), Xaa(120), Xaa(156), Serin, Tyrosin oder Theronin sind und für die anderen Aminosäuren gilt
Xaa(5) ist Leu, Xaa (8) ist Phe,
Xaa(15) ist Phe, Xaa(47) ist Leu,
Xaa(50) ist Phe, Xaa(106) ist Leu,
Xaa(111) ist Phe, Xaa(116) ist Leu,
Xaa(120) ist Leu, Xaa(156) ist Phe.

4. Interferon-β nach mindestens einem der vorhergehenden Ansprüche, **dadurch gekennzeichnet, daß** es die folgende Aminosäuresequenz enthält:

5. Verwendung von Interferon-β nach mindestens einem der vorhergehenden Ansprüche zur Herstellung eines Arzneimittels, als Feinchemikalie für invitro-Versuche oder für Interferonspiegelmessungen.

6. Herstellungsverfahren für ein humanes, rekombinantes Interferon-β nach Anspruch 1 unter Verwendung eines Mikroorganismus, der das Gen für rekombinantes humanes Interferon-β enthält,
**dadurch gekennzeichnet,**
**daß** in dem Gen für humanes, rekombinantes Interferon-β des Organismus der genetische Code für mindestens eine der Aminosäuren Leu(5), Phe(8), Phe(15), Leu(47), Phe(50), Leu(106), Phe(111), Leu(116), Leu(120), Phe(156) gegen einen genetischen Code für Aminosäuren mit mindestens einer Hydroxygruppe, vorzugsweise Serin, Tyrosin oder Threonin, ausgetauscht wird.

7. Herstellungsverfahren für ein humanes, rekombinantes Interferon-β nach Anspruch 1 unter Verwendung eines Mikroorganismus,
**dadurch gekennzeichnet,**
**daß** in das genetische Material des Mikroorganismus das Gen für humanes, rekombinantes Interferon-β eingefügt wird, wobei der genetische Code für mindestens eine der Aminosäuren Leu(5), Phe(8), Phe(15), Leu(47), Phe(50), Leu(106), Phe(111) , Leu(116), Leu(120), Phe(156) des eingefügten Gens gegen einen genetischen Code für Aminosäuren mit mindestens einer Hydroxygruppe, vorzugsweise Serin, Tyrosin oder Threonin, ausgetauscht ist.

## Claims

1. Human, recombinant β-interferon,
***characterised in that***
at least one of the amino acids Leu (5), Phe (8), Phe (15), Leu (47), Phe (50), Leu (106), Phe (111), Leu (116), Leu (120), Phe (156) is exchanged for amino acids with at least one hydroxy group.

2. β-interferon according to claim 1, **characterised in that** the amino acids with hydroxy groups are serine, tyrosine and/or threonine.

3. β-interferon according to at least one of the preceding claims, **characterised in that** it contains the following amino-acid sequence: one or more of the amino acids Xaa (5), Xaa (8), Xaa (15), Xaa (47), Xaa (50), Xaa (106), Xaa (111), Xaa (116), Xaa (120), Xaa (156) being serine, tyrosine or threonine and there applying for the other amino acids
Xaa (5) is Leu, Xaa (8) is Phe,
Xaa (15) is Phe, Xaa (47) is Leu,
Xaa (50) is Phe, Xaa (106) is Leu,
Xaa (111) is Phe, Xaa (116) is Leu
if Xaa (120) is Leu, Xaa (156) is Phe.

4. β-interferon according to at least one of the preceding claims, **characterised in that** it contains the following amino-acid sequence:

5. Use of β-interferon according to at least one of the preceding claims for producing a medicine, as a fine chemical for in vitro tests or for interferon level measurements.

6. Production method for a human, recombinant β-interferon according to claim 1 using a microorganism which contains the gene for recombinant human β-interferon,
***characterised in that***
in the gene for human recombinant β-interferon of the organism, the genetic code for at least one of the amino acids Leu (5), Phe (8), Phe (15), Leu (47), Phe (50), Leu (106), Phe (111), Leu (116), Leu (120), Phe (156) is exchanged for a genetic code for amino acids with at least one hydroxy group, preferably serine, tyrosine or threonine.

7. Production method for a human, recombinant β-interferon according to claim 1 using a microorganism,
***characterised in that***
the gene for human, recombinant β-interferon is introduced into the genetic material of the microorganism, the genetic code for at least one of the amino acids Leu (5), Phe (8), Phe (15), Leu (47), Phe (50), Leu (106), Phe (111), Leu (116), Leu (120), Phe (156) of the introduced gene being exchanged for a genetic code for amino acids with at least one hydroxy group, preferably serine, tyrosine or threonine.

## Revendications

1. Interféron-bêta humain recombinant
**caractérisé en ce qu'**
au moins l'un des acides aminés Leu(5), Phe(8), Phe(15), Leu(47), Phe(50), Leu( 106), Phe(111), Leu( 116), Leu(120), Phe(156) est remplacé par des acides aminés comportant au moins un groupe hydroxy.

2. Interféron-bêta selon la revendication 1,
**caractérisé en ce que**
les acides aminés comportant des groupes hydroxy sont la sérine, la tyrosine et/ou la thréonine.

3. Interféron-bêta selon au moins l'une quelconque des revendications précédentes,
**caractérisé en ce qu'**
il contient la séquence d'acides aminés suivante : dans laquelle un ou plusieurs des acides aminés Xaa(5), Xaa(8), Xaa(15), Xaa(47), Xaa(50), Xaa(106), Xaa(111), Xaa(116), Xaa(120), Xaa(156), sont la sérine, la tyrosine ou la thréonine et pour les autres acides aminés,
Xaa(5) représente Leu, Xaa(8) représente Phe,
Xaa(15) représente Phe, Xaa(47) représente Leu,
Xaa(50) représente Phe, Xaa( 106) représente Leu,
Xaa(111) représente Phe, Xaa(116) représente Leu,
Xaa(120) représente Leu, Xaa(156) représente Phe

4. Interféron-bêta selon au moins l'une quelconque des revendications précédentes,
**caractérisé en ce qu'**
il contient la séquence d'acides aminés suivante :

5. Utilisation d'interféron-bêta selon au moins l'une quelconque des revendications précédentes pour la fabrication d'un médicament, en tant que produit chimique fin pour des essais in vitro ou pour des mesures du taux d'interféron.

6. Procédé de fabrication d'un interféron-bêta humain recombinant selon la revendication 1 avec utilisation d'un microorganisme qui contient le gène de l'interféron-bêta humain recombinant,
**caractérisé en ce que**
dans le gène de l'interféron-bêta humain recombinant de l'organisme, le code génétique d'au moins un des acides aminés Leu(5), Phe(8), Phe(15), Leu(47), Phe(50), Leu(106), Phe(111), Leu(116), Leu(120), Phe(156) est remplacé par un code génétique d'acides aminés contenant au moins un groupe hydroxy, de préférence de la sérine, de la tyrosine ou de la thréonine.

7. Procédé de fabrication d'un interféron-bêta humain recombinant selon la revendication 1 avec utilisation d'un micro-organisme,
**caractérisé en ce que**
l'on introduit dans le matériel génétique du micro-organisme le gène de l'interféron-bêta humain recombinant, dans lequel le code génétique d'au moins un des acides aminés Leu(5), Phe(8), Phe(15), Leu(47), Phe(50), Leu(106), Phe(111), Leu(116), Leu(120), Phe(156) du gène introduit est remplacé par un code génétique d'acides aminés contenant au moins un groupe hydroxy, de préférence de la sérine, de la tyrosine ou de la thréonine.


### SEQUENZPROTOKOLL

(1) ALLGEMEINE ANGABEN:
   (i) ANMELDER:
      (A) NAME: Fraunhofer-Gesellschaft zur Förderung der angewandten Forschung e. V.
      (B) STRASSE: Leonrodstrasse 54
      (C) ORT: München
      (D) BUNDESLAND: Freistaat Bayern
      (E) LAND: Bundesrepublik Deutschland
      (F) POSTLEITZAHL: 80636
   (ii) BEZEICHNUNG DER ERFINDUNG: Humanes rekombinantes Interferon-beta mit verbesserter Löslichkeit
   (iii) ANZAHL DER SEQUENZEN: 22
   (iv) COMPUTER-LESBARE FASSUNG:
      (A) DATENTRÄGER: Diskette
      (B) COMPUTER: IBM PC-kompatibel
      (C) BETRIEBSSYSTEM: PC-DOS/MS-DOS
      (D) SOFTWARE: PatentIn (EPA)
(2) ANGABEN ZU SEQ ID NO: 1:
   (i) SEQUENZKENNZEICHEN:
      (A) LÄNGE: 166 Aminosäuren
      (B) ART: Aminosäure
      (D) TOPOLOGIE: linear
   (ii) ART DES MOLEKÜLS: Protein
   (iii) HYPOTHETISCH: NEIN
   (vi) URSPRÜNGLICHE HERKUNFT:
      (A) ORGANISMUS: Homo sapiens
      (G) ZELLTYP: Fibroblast
   (xi) SEQUENZBESCHREIBUNG: SEQ ID NO: 1:
   Met Ser Tyr Asn Leu Leu Gly Phe Leu Gln Arg Ser Ser Asn Phe Gln
   1               5                   10                  15
   Cys Gln Lys Leu Leu Trp Gln Leu Asn Gly Arg Leu Glu Tyr Cys Leu
               20                  25                  30
   Lys Asp Arg Met Asn Phe Asp Ile Pro Glu Glu Ile Lys Gln Leu Gln
           35                  40                  45
   Gln Phe Gln Lys Glu Asp Ala Ala Leu Thr Ile Tyr Glu Met Leu Gln
       50                  55                  60
   Asn Ile Phe Ala Ile Phe Arg Gln Asp Ser Ser Ser Thr Gly Trp Asn
   65                  70                  75                  80
   Glu Thr Ile Val Glu Asn Leu Leu Ala Asn Val Tyr His Gln Ile Asn
                   85                  90                  95
   His Leu Lys Thr Val Leu Glu Glu Lys Leu Glu Lys Glu Asp Phe Thr
               100                 105                 110
   Arg Gly Lys Leu Met Ser Ser Leu His Leu Lys Arg Tyr Tyr Gly Arg
           115                 120                 125
   Ile Leu His Tyr Leu Lys Ala Lys Glu Tyr Ser His Cys Ala Trp Thr
       130                 135                 140
   Ile Val Arg Val Glu Ile Leu Arg Asn Phe Tyr Phe Ile Asn Arg Leu
   145                 150                 155                 160
   Thr Gly Tyr Leu Arg Asn
                   165
(2) ANGABEN ZU SEQ ID NO: 2:
   (i) SEQUENZKENNZEICHEN:
      (A) LÄNGE: 166 Aminosäuren
      (B) ART: Aminosäure
      (D) TOPOLOGIE: linear
   (ii) ART DES MOLEKÜLS: Protein
   (iii) HYPOTHETISCH: NEIN
   (vi) URSPRÜNGLICHE HERKUNFT:
      (A) ORGANISMUS: Homo sapiens
      (G) ZELLTYP: Fibroblast
   (ix) MERKMALE:
      (A) NAME/SCHLÜSSEL: spezielles Merkmal
      (B) LAGE: 5
      (C) SONSTIGE ANGABEN: /NOTE= Xaa ist Leu, Ser, Tyr oder Thr
   (ix) MERKMALE:
      (A) NAME/SCHLÜSSEL: spezielles Merkmal
      (B) LAGE: 8
      (C) SONSTIGE ANGABEN: /NOTE= Xaa ist Phe, Ser, Tyr oder Thr
   (ix) MERKMALE:
      (A) NAME/SCHLÜSSEL: spezielles Merkmal
      (B) LAGE: 15
      (C) SONSTIGE ANGABEN: /NOTE= Xaa ist Phe, Ser, Tyr oder Thr
   (ix) MERKMALE:
      (A) NAME/SCHLÜSSEL: spezielles Merkmal
      (B) LAGE: 47
      (C) SONSTIGE ANGABEN: /NOTE= Xaa ist Leu, Ser, Tyr oder Thr
   (ix) MERKMALE :
      (A) NAME/SCHLÜSSEL: spezielles Merkmal
      (B) LAGE: 50
      (C) SONSTIGE ANGABEN: /NOTE= Xaa ist Phe, Ser, Tyr oder Thr
   (ix) MERKMALE:
      (A) NAME/SCHLÜSSEL: spezielles Merkmal
      (B) LAGE: 106
      (C) SONSTIGE ANGABEN: /NOTE= Xaa ist Leu, Ser, Tyr oder Thr
   ( ix) MERKMALE:
      (A) NAME/SCHLÜSSEL: spezielles Merkmal
      (B) LAGE: 111
      (C) SONSTIGE ANGABEN: /NOTE= Xaa ist Phe, Ser, Tyr oder Thr
   (ix) MERKMALE:
      (A) NAME/SCHLÜSSEL: spezielles Merkmal
      (B) LAGE: 116
      (C) SONSTIGE ANGABEN: /NOTE= Xaa ist Leu, Ser, Tyr oder Thr
   (ix) MERKMALE:
      (A) NAME/SCHLÜSSEL: spezielles Merkmal
      (B) LAGE: 120
      (C) SONSTIGE ANGABEN: /NOTE= Xaa ist Leu, Ser, Tyr oder Thr
   (ix) MERKMALE:
      (A) NAME/SCHLÜSSEL: spezielles Merkmal
      (B) LAGE: 151
      (C) SONSTIGE ANGABEN: /NOTE= Xaa ist Leu, Ser, Tyr oder Thr
   (xi) SEQUENZBESCHREIBUNG: SEQ ID NO: 2:
   Met Ser Tyr Asn Xaa Leu Gly Xaa Leu Gln Arg Ser Ser Asn Xaa Gln
   1               5                   10                  15
   Cys Gln Lys Leu Leu Trp Gln Leu Asn Gly Arg Leu Glu Tyr Cys Leu
               20                  25                  30
   Lys Asp Arg Met Asn Phe Asp Ile Pro Glu Glu Ile Lys Gln Xaa Gln
           35                  40                  45
   Gln Xaa Gln Lys Glu Asp Ala Ala Leu Thr Ile Tyr Glu Met Leu Gln
       50                  55                  60
   Asn Ile Phe Ala Ile Phe Arg Gln Asp Ser Ser Ser Thr Gly Trp Asn
   65                  70                  75                  80
   Glu Thr Ile Val Glu Asn Leu Leu Ala Asn Val Tyr His Gln Ile Asn
                   85                  90                  95
   His Leu Lys Thr Val Leu Glu Glu Lys Xaa Glu Lys Glu Asp Xaa Thr
               100                 105                 110
   Arg Gly Lys Xaa Met Ser Ser Xaa His Leu Lys Arg Tyr Tyr Gly Arg
           115                 120                 125
   Ile Leu His Tyr Leu Lys Ala Lys Glu Tyr Ser His Cys Ala Trp Thr
       130                 135                 140
   Ile Val Arg Val Glu Ile Xaa Arg Asn Phe Tyr Phe Ile Asn Arg Leu
   145                 150                 155                 160
   Thr Gly Tyr Leu Arg Asn
                   165
(2) ANGABEN ZU SEQ ID NO: 3:
   (i) SEQUENZKENNZEICHEN:
      (A) LÄNGE: 166 Aminosäuren
      (B) ART: Aminosäure
      (D) TOPOLOGIE: linear
   (ii) ART DES MOLEKÜLS: Protein
   (iii) HYPOTHETISCH: NEIN
   (vi) URSPRÜNGLICHE HERKUNFT:
      (A) ORGANISMUS: Homo sapiens
      (G) ZELLTYP: Fibroblast
   (xi) SEQUENZBESCHREIBUNG: SEQ ID NO: 3:
   Met Ser Tyr Asn Ser Leu Gly Ser Leu Gln Arg Ser Ser Asn Ser Gln
   1               5                   10                  15
   Cys Gln Lys Leu Leu Trp Gln Leu Asn Gly Arg Leu Glu Tyr Cys Leu
               20                  25                  30
   Lys Asp Arg Met Asn Phe Asp Ile Pro Glu Glu Ile Lys Gln Ser Gln
           35                  40                  45
   Gln Ser Gln Lys Glu Asp Ala Ala Leu Thr Ile Tyr Glu Met Leu Gln
       50                  55                  60
   Asn Ile Phe Ala Ile Phe Arg Gln Asp Ser Ser Ser Thr Gly Trp Asn
   65                  70                  75                  80
   Glu Thr Ile Val Glu Asn Leu Leu Ala Asn Val Tyr His Gln Ile Asn
                   85                  90                  95
   His Leu Lys Thr Val Leu Glu Glu Lys Ser Glu Lys Glu Asp Ser Thr
               100                 105                 110
   Arg Gly Lys Ser Met Ser Ser Ser His Leu Lys Arg Tyr Tyr Gly Arg
           115                 120                 125
   Ile Leu His Tyr Leu Lys Ala Lys Glu Tyr Ser His Cys Ala Trp Thr
       130                 135                 140
   Ile Val Arg Val Glu Ile Leu Arg Asn Phe Tyr Ser Ile Asn Arg Leu
   145                 150                 155                 160
   Thr Gly Tyr Leu Arg Asn
                   165
(2) ANGABEN ZU SEQ ID NO: 4
   (i) SEQUENZKENNZEICHEN:
      (A) LÄNGE: 25 Basenpaare
      (B) ART: Nucleinsäure
      (C) STRANGFORM: Einzelstrang
      (D) TOPOLOGIE: Linear
   (ii) ART DES MOLEKÜLS: DNA
   (xi) SEQUENZBESCHREIBUNG:SEQ ID NO:4:
   C TCC CTT GGA TTC CTA CAA AGA AGC                                25
(2) ANGABEN ZU SEQ ID NO: 5
   (i) SEQUENZKENNZEICHEN:
      (A) LÄNGE: 25 Basenpaare
      (B) ART: Nucleinsäure
      (C) STRANGFORM: Einzelstrang
      (D) TOPOLOGIE: Linear
   (ii) ART DES MOLEKÜLS: DNA
   (xi) SEQUENZBESCHREIBUNG:SEQ ID NO:5:
   C TTG CTT GGA TCC CTA CAA AGA AGC 25
(2) ANGABEN ZU SEQ ID NO: 6
   (i) SEQUENZKENNZEICHEN:
      (A) LÄNGE: 28 Basenpaare
      (B) ART: Nucleinsäure
      (C) STRANGFORM: Einzelstrang
      (D) TOPOLOGIE: Linear
   (ii) ART DES MOLEKÜLS: DNA
   (xi) SEQUENZBESCHREIBUNG:SEQ ID NO:6:
   AGC AGC AAT TCT CAG TCC CAG AAG CTC C 28
(2) ANGABEN ZU SEQ ID NO: 7
   (i) SEQUENZKENNZEICHEN:
      (A) LÄNGE: 28 Basenpaare
      (B) ART: Nucleinsäure
      (C) STRANGFORM: Einzelstrang
      (D) TOPOLOGIE: Linear
   (ii) ART DES MOLEKÜLS: DNA
   (xi) SEQUENZBESCHREIBUNG:SEQ ID NO:7:
   AGC AGC AAT TTT CAG TCC CAG AAG CTC C 28
(2) ANGABEN ZU SEQ ID NO: 8
   (i) SEQUENZKENNZEICHEN:
      (A) LÄNGE: 27 Basenpaare
      (B) ART: Nucleinsäure
      (C) STRANGFORM: Einzelstrang
      (D) TOPOLOGIE: Linear
   (ii) ART DES MOLEKÜLS: DNA
   (xi) SEQUENZBESCHREIBUNG:SEQ ID NO:8:
   TT AAG CAG TCC CAG CAG TTC CAG AAG G 27
(2) ANGABEN ZU SEQ ID NO: 9
   (i) SEQUENZKENNZEICHEN:
      (A) LÄNGE: 27 Basenpaare
      (B) ART: Nucleinsäure
      (C) STRANGFORM: Einzelstrang
      (D) TOPOLOGIE: Linear
   (ii) ART DES MOLEKÜLS: DNA
   (xi) SEQUENZBESCHREIBUNG:SEQ ID NO:9:
   TT AAG CAG CTG CAG CAG TCC CAG AAG G 27
(2) ANGABEN ZU SEQ ID NO: 10
   (i) SEQUENZKENNZEICHEN:
      (A) LÄNGE: 30 Basenpaare
      (B) ART: Nucleinsäure
      (C) STRANGFORM: Einzelstrang
      (D) TOPOLOGIE: Linear
   (ii) ART DES MOLEKÜLS: DNA
   (xi) SEQUENZBESCHREIBUNG:SEQ ID NO:10:
   GAA GAA AAA TCC GAG AAA GAA GAT TTC ACC 30
(2) ANGABEN ZU SEQ ID NO: 11
   (i) SEQUENZKENNZEICHEN:
      (A) LÄNGE: 30 Basenpaare
      (B) ART: Nucleinsäure
      (C) STRANGFORM: Einzelstrang
      (D) TOPOLOGIE: Linear
   (ii) ART DES MOLEKÜLS: DNA
   (xi) SEQUENZBESCHREIBUNG:SEQ ID NO:11:
   GAA GAA AAA CTG GAG AAA GAA GAT TCC ACC 30
(2) ANGABEN ZU SEQ ID NO: 12
   (i) SEQUENZKENNZEICHEN:
      (A) LÄNGE: 25 Basenpaare
      (B) ART: Nucleinsäure
      (C) STRANGFORM: Einzelstrang
      (D) TOPOLOGIE: Linear
   (ii) ART DES MOLEKÜLS: DNA
   (xi) SEQUENZBESCHREIBUNG:SEQ ID NO:12:
   A AAA TCC ATG AGC AGT CTG CAC CTG 25
(2) ANGABEN ZU SEQ ID NO: 13
   (i) SEQUENZKENNZEICHEN:
      (A) LÄNGE,: 25 Basenpaare
      (B) ART: Nucleinsäure
      (C) STRANGFORM: Einzelstrang
      (D) TOPOLOGIE: Linear
   (ii) ART DES MOLEKÜLS: DNA
   (xi) SEQUENZBESCHREIBUNG:SEQ ID NO:13:
(2) ANGABEN ZU SEQ ID NO: 14
   (i) SEQUENZKENNZEICHEN:
      (A) LÄNGE: 28 Basenpaare
      (B) ART: Nucleinsäure
      (C) STRANGFORM: Einzelstrang
      (D) TOPOLOGIE: Linear
   (ii) ART DES MOLEKÜLS: DNA
   (xi) SEQUENZBESCHREIBUNG:SEQ ID NO:14:
   AC TTT TAC TCC ATT AAC AGA CTT ACA GG 28
(2) ANGABEN ZU SEQ ID NO: 15
   (i) SEQUENZKENNZEICHEN:
      (A) LÄNGE: 25 Basenpaare
      (B) ART: Nucleinsäure
      (C) STRANGFORM: Einzelstrang
      (D) TOPOLOGIE: Linear
   (ii) ART DES MOLEKÜLS: DNA
   (xi) SEQUENZBESCHREIBUNG:SEQ ID NO:15:
   TT GTA GCT CAT ATG TAA GTA TTT CC 25
(2) ANGABEN ZU SEQ ID NO: 16
   (i) SEQUENZKENNZEICHEN:
      (A) LÄNGE: 29 Basenpaare
      (B) ART: Nucleinsäure
      (C) STRANGFORM: Einzelstrang
      (D) TOPOLOGIE: Linear
   (ii) ART DES MOLEKÜLS: DNA
   (xi) SEQUENZBESCHREIBUNG:SEQ ID NO:16:
   TCT TTG TAG GAA TCC AAG CAA GTT GTA GC 29
(2) ANGABEN ZU SEQ ID NO: 17
   (i) SEQUENZKENNZEICHEN:
      (A) LÄNGE: 27 Basenpaare
      (B) ART: Nucleinsäure
      (C) STRANGFORM: Einzelstrang
      (D) TOPOLOGIE: Linear
   (ii) ART DES MOLEKÜLS: DNA
   (xi) SEQUENZBESCHREIBUNG:SEQ ID NO:17:
   T CTC CTC AGG GAT GTC AAA GTT CAT CC 27
(2) ANGABEN ZU SEQ ID NO: 18
   (i) SEQUENZKENNZEICHEN:
      (A) LÄNGE: 27 Basenpaare
      (B) ART: Nucleinsäure
      (C) STRANGFORM: Einzelstrang
      (D) TOPOLOGIE: Linear
   (ii) ART DES MOLEKÜLS: DNA
   (xi) SEQUENZBESCHREIBUNG:SEQ ID NO:18:
   CAG GAC TGT CTT CAG ATG GTT TAT CTG 27
(2) ANGABEN ZU SEQ ID NO: 19
   (i) SEQUENZRENNZEICHEN:
      (A) LÄNGE: 23 Basenpaare
      (B) ART: Nucleinsäure
      (C) STRANGFORM: Einzelstrang
      (D) TOPOLOGIE: Linear
   (ii) ART DES MOLEKÜLS: DNA
   (xi) SEQUENZBESCHREIBUNG:SEQ ID NO:19:
   CC CCT GGT GAA ATC TTC TTT CTC 23
(2) ANGABEN ZU SEQ ID NO: 20
   (i) SEQUENZKENNZEICHEN:
      (A) LÄNGE: 27 Basenpaare
      (B) ART: Nucleinsäure
      (C) STRANGFORM: Einzelstrang
      (D) TOPOLOGIE: Linear
   (ii) ART DES MOLEKÜLS: DNA
   (xi) SEQUENZBESCHREIBUNG:SEQ ID NO:20:
   r TCT TAG GAT TTC CAC TCT GAC TAT GG 27
(2) ANGABEN ZU SEQ ID NO: 21
   (i) SEQUENZKENNZEICHEN:
      (A) LÄNGE: 29 Basenpaare
      (B) ART: Nucleinsäure
      (C) STRANGFORM: Einzelstrang
      (D) TOPOLOGIE: Linear
   (ii) ART DES MOLEKÜLS: DNA
   (xi) SEQUENZBESCHREIBUNG:SEQ ID NO:21:
   TCT TTG TAG GGA TCC AAG GGA GTT GTA GC 29
(2) ANGABEN ZU SEQ ID NO: 22
   (i) SEQUENZKENNZEICHEN:
      (A) LÄNGE: 26 Basenpaare
      (B) ART: Nucleinsäure
      (C) STRANGFORM: Einzelstrang
      (D) TOPOLOGIE: Linear
   (ii) ART DES MOLEKÜLS: DNA
   (xi) SEQUENZBESCHREIBUNG:SEQ ID NO:22:
   CC CCT GGT GGA ATC TTC TTT CTC GGA 26